# FASCICULE DE BREVET EUROPEEN

(11) **EP 0 788 361 B1**
(45) Date de publication et mention de la délivrance du brevet: **05.06.2002**
(21) Numéro de dépôt: 96931100.0
(22) Date de dépôt: 10.09.1996
(51) Int. Cl.: A61K 31/59

(54) **NOUVELLES COMPOSITIONS A BASE D'UN MELANGE SYNERGETIQUE ENTRE AU MOINS UN LIGAND DE VDR ET UN RETINOIDE**
NEUARTIGE ZUSAMMENSETZUNG BASIEREND AUF EINER SYNERGISTISCHEN MISCHUNG VON EINEM ODER MEHREREN VRD-LIGANDEN UND RETINOIDEN
NOVEL COMPOSITIONS BASED ON A SYNERGISTIC COMBINATION OF ONE OR MORE VRD LIGANDS AND RETINOIDS

(30) Priorité: 15.09.1995 FR 9510854
(43) Date de publication de la demande: 13.08.1997
(73) Titulaire: CENTRE INTERNATIONAL DE RECHERCHES DERMATOLOGIQUES GALDERMA (C.I.R.D. GALDERMA), 06560 Valbonne (FR)
(72) Inventeur: MICHEL, Serge, F-06330 Roquefort-les-Pins (FR); DIONISIUS-POUGET, Véronique, F-06560 Valbonne (FR)
(74) Mandataire: Tezier Herman, Béatrice
(86) Numéro de dépôt international: FR9601386
(87) Numéro de publication internationale: WO9709987

(56) Documents cités:
- EP-A- 0 658 553
- DATABASE EMBASE ELSEVIER SCIENCE PUBLISHERS, AMSTERDAM, NL TRAFNA R. ET AL: "Biologic basis of retinoids and vitamin D3 action in proliferative skin diseases" XP002023091 & PRZEGL. DERMATOL., 1994, 81/6 (573-578), POLAND,
- JOURNAL OF INVESTIGATIVE DERMATOLOGY, 104 (4). 1995. 604., XP000573627 MAJEWSKI S ET AL: "Retinoids and 1,25-dihydroxyvitamin D3 (VD3) synergistically inhibit angiogenic potential of human transformed epithelial cell lines"
- LEUKEMIA (BASINGSTOKE), 8 (SUPPL. 3). 1994. S11-S15., XP000573670 BOLLAG W ET AL: "Cancer combination chemotherapy with retinoids: Experimental rationale"
- CANCER LETTERS, 75 (1). 1993. 35-39., XP000574177 MAJEWSKI S ET AL: "Inhibition of tumor cell-induced angiogenesis by retinoids, 1,25-dihydroxyvitamin D-3 and their combination"

## Description

L'invention concerne une nouvelle composition, notamment pharmaceutique, comprenant une association synergétique entre au moins un ligand présentant une activité pour les récepteurs nucléaires de type VDR et au moins un rétinoïde particulier, ainsi que l'utilisation de cette composition dans le domaine pharmaceutique ou cosmétique.

On sait qu'un ligand présentant une activité pour les récepteurs nucléaires de type VDR (récepteur de la vitamine D3), tel que la 1α,25-dihydroxyvitamine D3 ou ses analogues, inhibe la prolifération des kératinocytes. Ainsi, ce type de composé est utilisé pour traiter le psoriasis. Cependant, ces composés, tels que la 1α,25-dihydroxyvitamine D3, présentent des effets secondaires : l'hypercalcémie ou hypercalciurie, dues à une augmentation du taux de calcium sérique.

On sait que l'acide rétinoïque est un modulateur (i.e. un inhibiteur ou, au contraire, un stimulateur, selon la nature des cellules traitées) de la différenciation et/ou de la prolifération de nombreux types cellulaires normaux ou transformés. Par exemple, il inhibe la différenciation des cellules épithéliales, tels que les kératinocytes de l'épiderme, sans être spécialement actif sur la prolifération de ces cellules. Il inhibe aussi la prolifération de nombreuses cellules transformées, telles que les cellules de mélanomes.

On sait, d'une manière générale, que l'acide rétinoïque tout-trans (all-trans retinoic acid) agit sur la différenciation et/ou la prolifération des cellules en interagissant avec des récepteurs nucléaires ou RARs (Retinoic Acid Receptors) contenus dans le noyau cellulaire. De nombreux analogues structuraux synthétiques de l'acide rétinoïque tout-trans ou de l'acide 9-cis-rétinoïque, couramment dénommés "rétinoïdes", ont été décrits à ce jour dans la littérature. Il existe, à ce jour, trois sous-types identifiés de récepteurs RAR appelés respectivement RAR-α, RAR-β et RAR-γ. Ces récepteurs, aprés fixation du ligand (i.e. de l'acide rétinoïque tout-trans), interagissent avec la région promotrice de gènes régulés par l'acide rétinoïque au niveau d'éléments de réponses spécifiques (RARE).

Certains analogues peuvent se fixer et activer un sous-type de récepteur RAR (α, β ou γ) particulier. D'autres analogues, enfin, ne présentent aucune activité sélective particulière vis-à-vis de ces différents récepteurs. A cet égard, et par exemple, l'acide rétinoïque tout-trans active les RARs (ligand agoniste spécifique RARs), tous sous-types confondus alors que l'acide rétinoïque 13-cis ne se lie pas aux récepteurs α ou γ.

Certains de ces rétinoïdes ont déjà été associés avec des dérivés de la vitamine D. Ainsi, dans la demande de brevet AU-A-37161/93, l'acide 9-cis- ou 13-cis-rétinoïque est associé avec des dérivés de la vitamine D. Cependant, ces associations s'avèrent peu satisfaisantes notamment dans le traitement du psoriasis. On comprend donc la nécessité de trouver des associations particulières présentant une efficacité remarquable, notamment dans le traitement du psoriasis.

Un des buts de la présente invention est donc de pouvoir disposer d'un nouveau produit présentant une inhibition remarquable de la prolifération cellulaire, en particulier des cellules de la peau, et plus particulièrement des kératinocytes, permettant à ce produit d'être utilisée dans le traitement des désordres liés à une hyperprolifération cellulaire, en particulier des désordres dermatologiques liés à une hyperprolifération cellulaire.

Un autre but de la présente invention est de pouvoir disposer d'un médicament diminuant, voire supprimant, les effets secondaires des ligands présentant une activité pour les récepteurs nucléaires de type VDR.

La Demanderesse vient maintenant de découvrir que l'association, nouvelle en soi, et notamment à titre de médicament, d'au moins un ligand présentant une activité pour les récepteurs nucléaires de type VDR et un rétinoïde particulier qui est sélectif des récepteurs RARγ par rapport aux récepteurs RARα choisi parmi l'acide 6-[3-(1-adamantyl)-4-hydroxyphényl]-2-naphtanoïque, l'acide 6-[3-(1-adamantyl)-4-méthoxyphényl]-2-naphtanoïque, l'acide 2-hydroxy -4-[3-hydroxy -3-(5,6,7,8-tétrahydro-5,5,8,8-tétraméthyl -2-naphtyl) -1-propynyl] benzoïque, ou leurs dérivés, permettait d'inhiber de manière tout à fait remarquable la prolifération des kératinocytes. Ce résultat est d'autant plus inattendu et surprenant que ces rétinoïdes, lorsqu'ils sont utilisés seuls, ne présentent aucune, ou substantiellement aucune, activité antiproliférative propre vis-à-vis de ces mêmes cellules.

Cette découverte est à la base de la présente invention.

Cette association conforme à l'invention trouve naturellement, compte tenu des activités remarquables qu'elle présente vis-à-vis des kératinocytes, une application privilégiée dans le traitement des désordres dermatologiques liés à une hyperprolifération des cellules de la peau, et plus particulièrement des kératinocytes.

Ainsi, dans l'un de ses premiers aspects, la présente invention a pour objet un produit de combinaison constitué par l'association d'au moins un ligand présentant une activité pour les récepteurs nucléaires de type VDR et d'au moins un rétinoïde sélectif des récepteurs RARγ par rapport aux récepteurs RARα choisi parmi l'acide 6-[3-(1 adamantyl)-4-hydroxyphényl]-2-naphtanoïque, l'acide 6-[3-(1-adamantyl)-4-méthoxyphényl]-2-naphtanoïque, l'acide 2-hydroxy -4-[3-hydroxy -3-(5,6,7,8-tétrahydro-5,5,8,8-tétraméthyl-2-naphtyl)-1-propynyl] benzoïque, ou leurs dérivés.

D'une manière générale et qualitativement, une substance donnée (ou ligand) est dite spécifique d'un récepteur particulier lorsque ladite substance présente une affinité pour ledit récepteur particulier plus forte que celle qu'elle présente par ailleurs pour les autres récepteurs.

Les constantes de dissociation sont déterminées au moyen de tests classiques pour l'homme de l'art. Ces tests sont notamment décrits dans les références suivantes : (1) "Selective Synthetic Ligands for Nuclear Retinoic Acid Receptor Subtypes" in RETINOIDS, Progress in Research and Clinical Applications, Chapitre 19 (pp 261-267), Marcel Dekker Inc, édité par Maria A. Livrea et Lester Packer ; (2) "Synthetic Retinoids : Receptor Selectivity and Biological Activity" in Pharmacol Skin, Basel, Karger, 1993, Volume. 5, pp 117-127 ; (3) "Selective Synthetic Ligands for Human Nuclear Retinoic Acid Receptors" in Skin Pharmacology, 1992, Vol. 5, pp 57-65 ; (4) "Identification of Synthetic Retinoids with Selectivity for Human Nuclear Retinoic Acid Receptor-γ" in Biochemical and Biophysical Research Communications, Vol. 186, N° 2, Juillet 1992, pp 977-983 ; (5) "Selective High Affinity RAR-α or RAR-β Retinoic Acid Receptor Ligands" in Mol. Pharmacol., Vol 40, pp 556-562.

D'une manière avantageuse, les rétinoïdes sélectifs des récepteurs RARγ par rapport aux récepteurs RARα, présentent un rapport de constantes de dissociation RARα /RARγ supérieur ou égal à 8.

Parmi les rétinoïdes sélectifs des récepteurs RARγ par rapport aux récepteurs RARα, on peut citer en particulier l'acide 6-[3-(1-adamantyl) -4-hydroxyphényl] -2-naphtanoïque (RARα / RARγ = 8,5) , l'acide 6-[3-(1-adamantyl) -4-méthoxyphényl] -2-naphtanoïque (RARα / RARγ = 84,4), l'acide 2-hydroxy -4-[3-hydroxy -3-(5,6,7,8-tétrahydro -5,5,8,8-tétraméthyl -2-naphtyl) -1-propynyl] benzoïque (RARα / RARγ = 36,6) ou leurs dérivés.

D'une manière générale, le rapport pondéral entre l'au moins un ligand présentant une activité pour les récepteurs nucléaires de type VDR et l'au moins un rétinoïde sélectif des récepteurs RARγ par rapport aux récepteurs RARα est compris entre 1/1000 et 1000/1. De préférence, ce rapport pondéral est compris entre 1/10 et 10/1.

La présente invention a également pour objet une nouvelle composition, notamment pharmaceutique, caractérisée par le fait qu'elle comprend, dans un support physiologiquement acceptable, un produit de combinaison tel que défini ci-dessus.

Les ligands présentant une activité pour le récepteur VDR sont utilisés pour le traitement de l'ostéoporose, il est donc envisageable d'utiliser ce produit de combinaison selon l'invention pour le traitement de l'ostéoporose.

Cependant, ce produit de combinaison s'avère très intéressant pour un traitement destiné à inhiber la prolifération cellulaire, en particulier des cellules de la peau, et plus particulièrement des kératinocytes, notamment pour le traitement du psoriasis.

L'invention a donc pour objet l'utilisation d'au moins un rétinoïde sélectif des récepteurs RARγ par rapport aux récepteurs RARα choisi parmi l'acide 6-[3-(1-adamantyl)-4-hydroxyphényl]-2-naphtanoïque, l'acide 6-[3-(1-adamantyl)-4-méthoxyphényl]-2-naphtanoïque, l'acide 2-hydroxy -4-[3-hydroxy-3-(5,6,7,8-tétrahydro-5,5,8,8-tétraméthyl -2-naphtyl) -1-propynyl] benzoïque, ou leurs dérivés, pour la fabrication d'une composition pharmaceutique destinée à augmenter l'activité inhibitrice de prolifération cellulaire, en particulier de cellules de la peau, plus particulièrement des kératinocytes, due à au moins un ligand présentant une activité pour le récepteur VDR utilisé pour le traitement des désordres dermatologiques liés à une hyperprolifération des cellules de la peau.

Ainsi, dans le cadre de cette utilisation selon l'invention, le rétinoïde peut être administré, de manière simultanée ou non, par des voies identiques ou non à l'administration du ligand présentant une activité pour le récepteur VDR.

La présente invention concerne donc également un produit comprenant au moins un ligand présentant une activité pour les récepteurs nucléaires de type VDR et au moins un rétinoïde sélectif des récepteurs RARγ par rapport aux récepteurs RARα choisi parmi l'acide 6-[3-(1-adamantyl)-4-hydroxyphényl]-2-naphtanoïque, l'acide 6-[3-(1-adamantyl)-4-méthoxyphényl]-2-naphtanoïque, l'acide 2-hydroxy -4-[3-hydroxy -3-(5,6,7,8-tétrahydro -5,5,8,8-tétraméthyl -2-naphtyl) -1-propynyl] benzoïque, ou leurs dérivés, comme produit de combinaison pour une utilisation simultanée, séparée ou étalée dans le temps pour le traitement des désordres dermatologiques liés à une hyperprolifération des cellules de la peau, plus particulièrement des kératinocytes.

Dans le cas où le rétinoïde est administré de manière simultanée et donc par une voie identique à l'administration du ligand présentant une activité pour le récepteur VDR, l'invention a enfin pour objet l'utilisation du produit de combinaison ci-dessus pour la fabrication d'une composition pharmaceutique, plus particulièrement dermatologique, destinée à traiter les désordres dermatologiques liés à une hyperprolifération des cellules de la peau, et plus particulièrement des kératinocytes. Cette composition est plus particulièrement destinée au traitement du psoriasis.

On comprend, dans les cellules de la peau, les kératinocytes, les mélanocytes, les fibroblastes, les cellules de Merkel et les cellules de Langerhans.

D'une manière générale, on notera que les dosés d'actifs à mettre en oeuvre pour obtenir l'effet recherché peuvent être faibles, ce qui constitue un avantage important lorsqu'il s'agit de faire face à des problèmes d'effets secondaires indésirables susceptibles d'apparaitre au sein des organismes à traiter ou en cours de traitement, tels que l'hypercalcémie ou l'hypercalciurie.

D'autres caractéristiques, aspects, objets et avantages de l'invention apparaîtront encore plus clairement à la lecture de la description et des figures qui vont suivre, ainsi que des divers exemples concrets, mais nullement limitatifs, destinés à l'illustrer.

### Description des figures :

**Figure 1**: Effet de l'association de la 1,25-dihydroxyvitamine D3 et du composé 1 (= l'acide 6-[3-(1-adamantyl)-4-hydroxyphényl]-2-naphtanoïque) sur l'inhibition de la prolifération des kératinocytes. Les résultats sont exprimés en pourcentage de prolifération des kératinocytes par rapport à un témoin (DMSO) en fonction de la concentration exprimée en nM de la 1,25-dihydroxyvitamine D3 en absence (□) ou en présence (■) du composé 1 à une concentration de 100 nM.
**Figure 2:** Effet de l'association de la 1,25-dihydroxyvitamine D3 et du composé 1 sur l'inhibition de la prolifération des kératinocytes. Les résultats sont expimés en pourcentage de prolifération des kératinocytes par rapport à un témoin (DMSO) en fonction de la concentration exprimée en nM du composé 1 en présence de 1 nM de 1,25-dihydroxyvitamine D3 (□). Sur cette figure, il est donné également la valeur du composé 1 seul à 100nM (■).

L'acide 6-[3-(1-adamantyl)-4-hydroxyphényl]-2-naphtanoïque et l'acide 6-[3-(1-adamantyl)-4-méthoxyphényl]-2-naphtanoïque ont déjà été décrits dans le brevet US 4,717,720. L'acide 2-hydroxy-4-[3-hydroxy-3-(5,6,7,8-tétrahydro-5,5,8,8-tétraméthyl-2-naphtyl)-1-propynyl]benzoïque est décrit dans la demande de brevet EP 661 258. Leurs dérivés peuvent correspondre à leurs esters, leurs amides, leurs sels, leurs alcools ou leurs aldéhydes.

Parmi les ligands présentant une activité pour le récepteur VDR, on peut citer les composés suivants :
- vitamine D3 (cholécalciférol),
- vitamine D2 (ergocalciférol),
- calcipotriol (ou calcipotriene), vendu notamment par la société LEO sous le nom commercial Daivonex®,
- 25-hydroxyvitamine D3,
- 1α-hydroxyvitamine D3,
- 1α,25-dihydroxyvitamine D3 (calcitriol),
- 1α,25,26-trihydroxyvitamine D3,
- 1α,23,25-trihydroxyvitamine D3,
- 24,25-dihydroxyvitamine D3,
- 1α,25-dihydroxyvitamine D2,
- 1α-hydroxyvitamine D2,
- 1α,24-dihydroxyvitamine D2,
- 1α,24-dihydroxyvitamine D3 (tacalcitol),
- le (5Z, 7E, 23S)-26,26,26,27,27,27-hexafluoro-9,10-secocholesta-5,7,10(19)-triène-1α-3β,23,25-tétraol,
- le 26,26,26,27,27,27-hexafluoro-9,10-secocholesta-5,7,10(19)-triène-1α,25-diol (26,26,26,27,27,27-hexafluoro-1α,25-dihydroxy-vitamine D3), ou
- les dérivés de la vitamine D décrits dans la demande de brevet WO 96/22776.

On préfère utiliser la 1α,25-dihydroxyvitamine D3.

Dans ce qui suit, on entend par voie topique, toute technique d'administration d'un produit par application directe de ce dernier sur une partie superficielle (ou externe) du corps, telle que la peau ou les muqueuses, et par voie systémique, toute technique d'administration d'un produit par une voie autre que topique, par exemple orale, entérale et/ou parentérale.

La présente invention a donc pour objet, entre autres, une nouvelle composition pharmaceutique destinée notamment au traitement de la peau, caractérisée par le fait qu'elle comprend, dans un support physiologiquement acceptable et compatible avec le mode d'administration retenu pour cette composition, au moins un ligand présentant une activité pour le récepteur VDR et au moins un rétinoïde sélectif des récepteurs RARγ par rapport aux récepteurs RARα choisi parmi l'acide 6-[3-(1 adamantyl)-4-hydroxyphényl]-2-naphtanoïque, l'acide 6-[3-(1-adamantyl)-4-méthoxyphényl]-2-naphtanoïque, l'acide 2-hydroxy -4-[3-hydroxy -3-(5,6,7,8-tétrahydro-5,5,8,8-tétraméthyl -2-naphtyl) -1-propynyl] benzoïque, ou leurs dérivés, à titre de principes actifs.

L'administration des actifs (rétinoïde ou ligand présentant une activité pour le récepteur VDR) ou de l'association selon l'invention peut être effectuée par voie entérale, parentérale, topique ou oculaire. Toutefois, de préférence, les compositions (ou associations) selon l'invention sont conditionnées sous une forme convenant à une application par voie topique.

Par voie entérale, les compositions peuvent se présenter sous forme de comprimés, de gélules, de dragées, de sirops, de suspensions, de solutions, de poudres, de granulés, d'émulsions, de microsphères ou de nanosphères ou de vésicules lipidiques ou polymériques permettant une libération contrôlée. Par voie parentérale, les compositions peuvent se présenter sous forme de solutions ou de suspensions pour perfusion ou pour injection.

Les mélanges d'actifs conformes à l'invention sont généralement administrés à des doses journalières d'environ 0,01 mg/kg à 100 mg/Kg en poids corporel, et ceci à raison de 1 à 3 prises/jour.

Par voie topique, les compositions pharmaceutiques, qui sont donc plus particulièrement destinées au traitement de la peau, peuvent se présenter sous forme d'onguents, de crèmes, de laits, de pommades, de poudres, de tampons imbibés, de solutions, de gels, de sprays, de lotions ou de suspensions. Elles peuvent également se présenter sous forme de microsphères ou nanosphères ou vésicules lipidiques ou polymériques ou de patches polymériques et d'hydrogels permettant une libération contrôlée des actifs. Ces compositions par voie topique peuvent par ailleurs se présenter soit sous forme anhydre, soit sous une forme aqueuse, selon l'indication clinique.

Les compositions à usage topique conformes à l'invention contiennent le ou les ligands de VDR à une concentration généralement comprise entre 0,001 % et 10 % en poids, de préférence entre 0,1 et 1 % en poids, par rapport au poids total de la composition. De même, les compositions à usage topique conformes à l'invention contiennent le ou les rétinoïdes à une concentration généralement comprise entre 0,001 % et 10 % en poids, de préférence entre 0,1 et 1 % en poids, par rapport au poids total de la composition.

Par voie oculaire, ce sont principalement des collyres.

Les compositions selon l'invention peuvent bien entendu en outre contenir des additifs inertes ou même pharmacodynamiquement actifs ou des combinaisons de ces additifs, et notamment : des agents mouillants; des agents dépigmentants tels que l'hydroquinone, l'acide azélaïque, l'acide caféïque ou l'acide kojique; des émollients; des agents hydratants comme le glycérol, le PEG 400, la thiamorpholinone, et ses dérivés ou bien encore l'urée; des agents antiséborrhéiques ou antiacnéiques, tels que la S-carboxyméthylcystéine, la S-benzyl-cystéamine, leurs sels ou leurs dérivés, ou le péroxyde de benzoyle; des agents antifongiques tels que le kétoconazole ou les polyméthylène-4,5 isothiazolidones-3; des antibactériens, des caroténoïdes et, notamment, le β-carotène; des agents anti-psoriatiques tels que l'anthraline et ses dérivés; et enfin les acides eicosa-5,8,11,14-tétraynoïque et eicosa-5,8,11-trynoïque, leurs esters et amides.

Les compositions selon l'invention peuvent également contenir des agents d'amélioration de la saveur, des agents conservateurs tels que les esters de l'acide parahydroxybenzoïque, les agents stabilisants, des agents régulateurs d'humidité, des agents régulateurs de pH, des agents modificateurs de pression osmotique, des agents émulsionnants, des filtres UV-A et UV-B, des antioxydants, tels que l'α-tocophérol, le butylhydroxyanisole ou le butylhydroxytoluène.

Les désordres dermatologiques liés à une hyperprolifération des cellules de la peau sont notamment les suivants :
1) Les affections dermatologiques liées à un désordre de la kératinisation portant sur la prolifération cellulaire notamment les acnés vulgaires, comédoniennes, polymorphes, rosacées, les acnés nodulokystiques, conglobata, les acnés séniles, les acnés secondaires telles que l'acné solaire, médicamenteuse ou professionnelle.
2) Les autres troubles de la kératinisation, notamment les ichtyoses, les états ichtyosiformes, la maladie de Darier, les kératodermies palmoplantaires, les leucoplasies et les états leucoplasiformes, le lichen cutané ou muqueux (buccal).
3) Les autres affections dermatologiques liées à un trouble de la kératinisation avec une composante inflammatoire et/ou immuno-allergique et, notamment, toutes les formes de psoriasis qu'il soit cutané, muqueux ou unguéal, et même le rhumatisme psoriatique, ou encore l'atopie cutanée, telle que l'eczéma ou l'atopie respiratoire ou encore l'hypertrophie gingivale.
4) Toutes les proliférations dermiques ou épidermiques qu'elles soient bénignes ou malignes, qu'elles soient ou non d'origine virale telles que verrues vulgaires, les verrues planes et l'épidermodysplasie verruciforme, les papillomatoses orales ou florides et les proliférations pouvant être induites par les ultra-violets notamment dans le cas des épithélioma baso et spinocellulaires.
5) D'autres désordres dermatologiques, tels que les dermatoses bulleuses et les maladies du collagène.
6) Le vieillissement de la peau, qu'il soit photo-induit ou chronologique ou pour les pigmentations et les kératoses actiniques, ou toutes pathologies associées au vieillissement chronologique ou actinique.
7) Les stigmates de l'atrophie épidermique et/ou dermique induite par les corticostéroïdes locaux ou systémiques, ou tout autre forme d'atrophie cutanée.
8) Les troubles de la cicatrisation ou les vergetures.
9) Les troubles de la fonction sébacée tels que l'hyperséborrhée de l'acné ou la séborrhée simple.
10) Les états cancéreux ou précancéreux de la peau.
11) Toute affection d'origine virale au niveau cutané.
12) Affections dermatologiques à composante immunologique.

La compositions selon l'invention s'avèrent particulièrement efficaces pour les traitements 1) à 4), et plus spécialement pour le traitement de toutes les formes de psoriasis.

On va maintenant donner, à titre nullement limitatif, plusieurs exemples destinés d'une part à démontrer les effets attachés à la présente invention, et, d'autre part, à illustrer diverses formulations concrètes conformes à l'invention.

### EXEMPLE 1

Cet exemple a pour but de mettre en évidence l'activité in vitro de l'association synergétique conforme à l'invention sur la prolifération des kératinocytes. Les kératinocytes ont été obtenues à partir de spécimen de peau provenant de chirurgie plastique. Ils sont utilisés au deuxième passage.

Le protocole expérimental et les méthodes de détermination de la prolifération étaient les suivants :

### Culture des kératinocytes :

Les cellules sont cultivées à 37°C dans une atmosphère humide en présence de 5% de CO₂ selon la méthode de Rheinwald et Green en présence de fibroblates 3T3 traités par la mitomycine dans du milieu MEM contenant 10 % (v/v) de sérum de veau foetal (SVF), 0,4 µg/ml d'hydrocortisone, 10ng/ml d'EGF (Epidermal growth factor) et 10⁻⁹M de chlolératoxine. Les cellules 3T3 sont ensemencées 24h avant les kératinocytes à raison de 15000 cellules par cm². Ensuite, les kératinocytes sont ensemmencées à raison de 4000 cellules par cm².

### Traitement des cellules :

Les cellules sont traitées 2h après l'ensemencement des kératinocytes avec le composé 1 ou la 1,25-diOHvitamine D3 dilués dans le DMSO. La concentration finale de DMSO dans le milieu de culture n'excède pas 0,2 % (v/v). Après 4 jours de culture, les cellules sont récoltées pour la détermination de la prolifération.

### Mesure de la prolifération :

La prolifération des cellules est déterminée au moyen d'un kit référencé "Cell proliferation kit II" (dosage XTT) mis en oeuvre selon les indications du fabriquant (Boehringer, ref. 1465 05). Elle est exprimée en unité d'absorbance à 495 nm. Plus l'absorbance mesurée est faible, plus la prolifération des kératinocytes a été inhibée.

Les résultats obtenus sont rassemblés dans les figures 1 et 2. Ces figures quantifient l'évolution du taux de la prolifération des kératinocytes en fonction de la concentration des actifs utilisés.

Ces figures montrent clairement que l'association du composé 1 et de la 1,25-dihydroxyvitamine D3 présente une très bonne inhibition sur la prolifération des kératinocytes.

La même expérience a été également réalisé avec une association de l'acide rétinoïque tout-trans, 9-cis ou 13-cis et de la 1,25-dihydroxyvitamine D3 dans des conditions indentiques. On observe aucune synergie d'inhibition sur la prolifération des kératinocytes.

### EXEMPLE 2

Dans cet exemple, on a illustré diverses formulations concrètes à base des associations conformes à l'invention (le composé 1 est celui défini ci-avant dans la description).

### A- VOIE ORALE

(a) Composition pour capsule molle de 100 mg
   - Composé 1 1,00 mg
   - 1α,25-dihydroxyvitamine D3 1,00 mg
   - BHT 0,01 mg
   - D,L-α-tocophérol 0,05 mg
   - Huile végétale qsp 100 mg
(b) Suspension buvable en ampoules de 10 ml
   - Composé 1 0,05 g
   - 1α,25-dihydroxyvitamine D3 0,05 g
   - Glycérine 1,000g
   - Sorbitol à 70% 1,000g
   - Saccharinate de sodium 0,010 g
   - Parahydroxybenzoate de méthyle 0,080 g
   - Arome qs
   - Eau purifiée qsp 10 ml

### B- VOIE TOPIQUE

(a) Onguent
   - Composé 1 0,1 g
   - 1α,25-dihydroxyvitamine D3 0,1 g
   - vaseline qsp 100
(b) Onguent
   - Composé 1 0,1 g
   - 1α,25-dihydroxyvitamine D3 0,1 g
   - Myristate d'isopropyle 81,520 g
   - Huile de vaseline fluide 9,100 g
   - Silice ("Aérosil 200" vendue par DEGUSSA) 9,180 g
(c) Crème Eau-dans-Huile non ionique
   - Composé 1 0,100 g
   - 1α,25-dihydroxyvitamine D3 0,100 g
   - Mélange d'alcools de lanoline émulsifs, de cires et d'huiles ("Eucerine anhydre" vendu par BDF) 39,900 g
   - Parahydroxybenzoate de méthyle 0,075 g
   - Parahydroxybenzoate de propyle 0,075 g
   - Eau déminéralisée stérile qsp 100 g
(d) Lotion
   - Composé 1 0,100 g
   - 1α,25-dihydroxyvitamine D3 0,100 g
   - Polyéthylène glycol (PEG 400) 69,800 g
   - Ethanol à 95% 30,000 g
(e) Onguent hydrophobe
   - Composé 1 0,300 g
   - 1α,25-dihydroxyvitamine D3 0,300 g
   - Mirystate d'isopropyle 36,400 g
   - Huile de silicone ("Rhodorsil 47 V 300" vendue par RHONE-POULENC) 36,400 g
   - Cire d'abeille 13,600 g
   - Huile de silicone ("DC200" 350 cps vendue par DOW CORNING) qsp 100g
(f) Crème Huile-dans-Eau non ionique
   - Composé 1 0,500 g
   - 1α,25-dihydroxyvitamine D3 0,500 g
   - Alcool cétylique 4,000 g
   - Monostéarate de glycérole 2,500 g
   - Stéarate de PEG 50 2,500 g
   - Beurre de karité 9,200 g
   - Propylène glycol 2,000 g
   - Parahydroxybenzoate de méthyle 0,075 g
   - Parahydroxybenzoate de propyle 0,075 g
   - Eau déminéralisée stérile qsp 100 g

## Revendications

1. Produit de combinaison constitué par l'association d'au moins un ligand présentant une activité pour les récepteurs nucléaires de type VDR et d'au moins un rétinoïde sélectif des récepteurs RARγ par rapport aux récepteurs RARα choisi parmi l'acide 6-[3-(1-adamantyl)-4-hydroxyphényl]-2-naphtanoïque, l'acide 6-[3-(1-adamantyl)-4-méthoxyphényl]-2-naphtanoïque, l'acide 2-hydroxy-4-[3-hydroxy-3-(5,6,7,8-tétrahydro-5,5,8,8-tétraméthyl-2-naphtyl)-1-propynyl] benzoïque, ou leurs dérivés.

2. Produit selon la revendication 1 pour une utilisation comme médicament.

3. Produit selon l'une des revendications 1 ou 2, **caractérisé en ce que** le rétinoïde sélectif des récepteurs RARγ par rapport aux récepteurs RARα, présente un rapport de constantes de dissociation RARα / RARγ supérieur ou égal à 8.

4. Produit selon l'une des revendications 1 à 3, **caractérisée en ce que** les ligands présentant une activité pour le récepteur VDR sont choisis parmi : la vitamine D3, la vitamine D2, la 25-hydroxyvitamine D3, la 1α-hydroxyvitamine D3, la 1α,25-dihydroxyvitamine D3 (calcitriol), la 1α,25,26-trihydroxyvitamine D3, la 1α,23,25-trihydroxyvitamine D3, la 24,25-dihydroxyvitamine D3, la 1α,25-dihydroxyvitamine D2, la 1α-hydroxyvitamine D2, la 1α,24-dihydroxyvitamine D2, la 1α,24-dihydroxyvitamine D3 (tacalcitol), le (5Z, 7E, 23S)-26,26,26,27,27,27-hexafluoro -9,10-secocholesta - 5,7,10(19)-triène -1α-3β,23,25-tétraol, et le 26,26,26,27,27,27-hexafluoro -9,10-secocholesta -5,7,10(19)-trièn e-1α,25-diol.

5. Produit selon la revendication 4, **caractérisé en ce que** les ligands présentant une activité pour le récepteur VDR est la 1α,25-dihydroxyvitamine D3.

6. Produit selon l'une des revendications 1 à 5, **caractérisé en ce que** le rapport pondéral entre l'au moins un ligand présentant une activité pour les récepteurs nucléaires de type VDR et l'au moins un rétinoïde sélectif des récepteurs RARγ par rapport aux récepteurs RARα est compris entre 1/1000 et 1000/1.

7. Produit selon la revendication 6, **caractérisé en ce que** le rapport pondéral est compris entre 1/10 et 10/1.

8. Composition pharmaceutique, **caractérisée en ce qu'**elle comprend, dans un support pharmaceutiquement acceptable, au moins un ligand présentant une activité pour les récepteurs nucléaires de type VDR et au moins un rétinoïde sélectif des récepteurs RARγ par rapport aux récepteurs RARα, tels que définis dans les revendications 1 à 7.

9. Composition selon la revendication 8, **caractérisée en ce qu'**il s'agit d'une composition conditionnée sous une forme convenant à un usage entérale, parentérale, topique ou oculaire.

10. Composition selon l'une des revendications 8 ou 9, **caractérisée en ce que** le ligand présentant une activité pour les récepteurs nucléaires de type VDR est présent à une concentration comprise entre 0,001 % et 10 % en poids, de préférence entre 0,1 et 1 % en poids, par rapport au poids total de la composition.

11. Composition selon l'une des revendications 8 à 10, **caractérisée en ce que** le rétinoïde est présent à une concentration comprise entre 0,001 % et 10 % en poids, de préférence entre 0,1 et 1 % en poids, par rapport au poids total de la composition.

12. Utilisation d'au moins un rétinoïde sélectif des récepteurs RARγ par rapport aux récepteurs RARα choisi parmi l'acide 6-[3-(1-adamantyl)-4-hydroxyphényl]-2-naphtanoïque, l'acide 6-[3-(1-adamantyl)-4-méthoxyphényl]-2-naphtanoïque, l'acide 2-hydroxy -4-[3-hydroxy -3-(5,6,7,8-tétrahydro -5,5,8,8-tétraméthyl -2-naphtyl) -1-propynyl] benzoïque, ou leurs dérivés pour la fabrication d'une composition pharmaceutique destinée à augmenter l'activité inhibitrice de prolifération cellulaire due à au moins un ligand présentant une activité pour le récepteur VDR utilisé pour le traitement des désordres liés à une hyperprolifération des cellules de la peau, et plus particulièrement des kératinocytes.

13. Utilisation du produit de combinaison tel que défini dans les revendications 1 à 7 pour la fabrication d'une composition pharmaceutique destinée à traiter les désordres liés à une hyperprolifération des cellules de la peau, et plus particulièrement des kératinocytes.

14. Utilisation selon l'une quelconque des revendications 12 à 13, **caractérisée en ce que** les désordres dermatologiques liés à une hyperprolifération des cellules de la peau sont choisis parmi les affections dermatologiques liées à un désordre de la kératinisation portant sur la prolifération cellulaire notamment les acnés vulgaires, comédoniennes, polymorphes, rosacées, les acnés nodulokystiques, conglobata, les acnés séniles, les acnés secondaires telles que l'acné solaire, médicamenteuse ou professionnelle, les autres troubles de la kératinisation, notamment les ichtyoses, les états ichtyosiformes, la maladie de Darier, les kératodermies palmoplantaires, les leucoplasies et les états leucoplasiformes, le lichen cutané ou muqueux (buccal), les autres affections dermatologiques liées à un trouble de la kératinisation avec une composante inflammatoire et/ou immuno-allergique et, notamment, toutes les formes de psoriasis qu'il soit cutané, muqueux ou unguéal, et même le rhumatisme psoriatique, ou encore l'atopie cutanée, telle que l'eczéma ou l'atopie respiratoire ou encore l'hypertrophie gingivale, toutes les proliférations dermiques ou épidermiques qu'elles soient bénignes ou malignes, qu'elles soient ou non d'origine virale telles que verrues vulgaires, les verrues planes et l'épidermodysplasie verruciforme, les papillomatoses orales ou florides et les proliférations pouvant être induites par les ultra-violets notamment dans le cas des épithélioma baso et spinocellulaires.

15. Produit comprenant au moins un ligand présentant une activité pour les récepteurs nucléaires de type VDR et au moins un rétinoïde sélectif des récepteurs RARγ par rapport aux récepteurs RARα, tels que définis dans les revendications 1 à 7, comme produit de combinaison pour une utilisation simultanée, séparée ou étalée dans le temps pour le traitement des désordres liés à une hyperprolifération cellulaire, en particulier les désordres dermatologiques liés à une hyperprolifération des cellules de la peau, plus particulièrement des kératinocytes.

## Patentansprüche

1. Kombinationsprodukt, das aus mindestens einem Liganden, der eine Wirksamkeit bzgl. der nukleären Rezeptoren vom VDR-Typ aufweist, in Kombination mit mindestens einem Retinoid, das im Vergleich mit RARα-Rezeptoren selektiv für RARγ-Rezeptoren ist, das unter der 6-[3-(1-Adamantyl)-4-hydroxyphenyl]-2-decahydronaphthalincarbonsäure, der 6-[3-(1-Adamantyl)-4-methoxyphenyl]-2-decahydronaphthalincarbonsäure, der 2-Hydroxy-4-[3-hydroxy-3-(5,6,7,8-tetrahydro-5,5,8,8-tetramethyl-2-naphthyl)-1-propinyl]-benzoesäure und den Derivaten dieser Verbindungen ausgewählt ist, besteht.

2. Produkt nach Anspruch 1 für eine Verwendung als Arzneimittel.

3. Produkt nach einem der Ansprüche 1 und 2, **dadurch gekennzeichnet, daß** das Retinoid, das im Vergleich mit RARα-Rezeptoren selektiv für RARγ-Rezeptoren ist, einen Quotienten der Dissoziationskonstanten RARα/RARγ von 8 oder darüber aufweist.

4. Produkt nach einem der Ansprüche 1 bis 3, **dadurch gekennzeichnet, daß** die Liganden, die eine Wirksamkeit bzgl. des VDR-Rezeptors aufweisen, ausgewählt sind unter: Vitamin D3, Vitamin D2, 25-Hydroxyvitamin D3, 1α-Hydroxyvitamin D3, 1α,25-Dihydroxyvitamin D3 (Calcitriol), 1α,25,26-Trihydroxyvitamin D3, 1α,23,25-Trihydroxyvitamin D3, 24,25-Dihydroxyvitamin D3, 1α,25-Dihydroxyvitamin D2, 1α-Hydroxyvitamin D2, 1α,24-Dihydroxyvitamin D2, 1α,24-Dihydroxyvitamin D3 (Tacalcitol), (5Z,7E, 23S)-26,26,26,27,27,27-Hexafluor-9,10-secocholesta-5,7,10(19)-trien-1α-3β,23,25-tetraol und 26,26,26,27,27,27-Hexafluor-9,10-secocholesta-5,7,10(19)-trien-1α,25-diol.

5. Produkt nach Anspruch 4, **dadurch gekennzeichnet, daß** es sich bei dem Liganden, der eine Wirksamkeit bzgl. des VDR-Rezeptors aufweist, um 1α,25-Dihydroxyvitamin D3 handelt.

6. Produkt nach einem der Ansprüche 1 bis 5, **dadurch gekennzeichnet, daß** das Gewichtsverhältnis des mindestens einen Liganden, der eine Wirksamkeit bzgl. der nukleären Rezeptoren vom VDR-Typ aufweist , zu dem mindestens einen Retinoid, das im Vergleich mit RARα-Rezeptoren selektiv für RARγ-Rezeptoren ist, im Bereich von 1/1000 bis 1000/1 liegt.

7. Produkt nach Anspruch 6, **dadurch gekennzeichnet, daß** das Gewichtsverhältnis im Bereich von 1/10 bis 10/1 liegt.

8. Pharmazeutische Zusammensetzung, **dadurch gekennzeichnet, daß** sie in einem pharmazeutisch akzeptablen Träger mindestens einen Liganden, der eine Wirksamkeit bzgl. der nukleären Rezeptoren vom VDR-Typ aufweist, und mindestens ein Retinoid, das im Vergleich mit RARα-Rezeptoren selektiv für RARγ-Rezeptoren ist, enthält, die wie in den Ansprüchen 1 bis 7 definiert sind.

9. Zusammensetzung nach Anspruch 8, **dadurch gekennzeichnet, daß** es sich um eine Zusammensetzung handelt, die in einer Form verpackt ist, die für eine enterale, parenterale, topische Verwendung oder eine Verwendung am Auge geeignet ist.

10. Zusammensetzung nach einem der Ansprüche 8 bis 10, **dadurch gekennzeichnet, daß** der Ligand, der eine Wirksamkeit bzgl. der nukleären Rezeptoren vom VDR-Typ aufweist, in einer Konzentration von 0,001 bis 10 Gew.-%, vorzugsweise 0,1 bis 1 Gew.-%, bezogen auf das Gesamtgewicht der Zusammensetzung, enthalten ist.

11. Zusammensetzung nach einem der Ansprüche 8 bis 10, **dadurch gekennzeichnet, daß** das Retinoid in einer Konzentration von 0,001 bis 10 Gew.-%, vorzugsweise 0,1 bis 1 Gew.-%, bezogen auf das Gesamtgewicht der Zusammensetzung, enthalten ist.

12. Verwendung mindestens eines Retinoids, das im Vergleich mit RARα-Rezeptoren selektiv für RARγ-Rezeptoren ist, das unter der 6-[3-(1-Adamantyl)-4-hydroxyphenyl]-2-decahydronaphthalincarbonsäure, der 6-[3-(1-Adamantyl)-4-methoxyphenyl]-2-decahydronaphthalincarbonsäure, der 2-Hydroxy-4-[3-hydroxy-3-(5,6,7,8-tetrahydro-5,5,8,8-tetramethyl-2-naphthyl)-1-propinyl]-benzoesäure und den Derivaten dieser Verbindungen ausgewählt ist, für die Herstellung einer pharmazeutischen Zusammensetzung, die dafür vorgesehen ist, die hemmende Wirkung auf die Zellproliferation zu verstärken, die durch mindestens einen Liganden verursacht wird, der eine Wirksamkeit bzgl. des VDR-Rezeptors aufweist, der für die Behandlung von Störungen verwendet wird, die mit einer übermäßigen Proliferation von Hautzellen und insbesondere der Keratinozyten verbunden sind.

13. Verwendung des Kombinationsprodukts, das wie in den Ansprüchen 1 bis 7 definiert ist, für die Herstellung einer pharmazeutischen Zusammensetzung, die für die Behandlung von Störungen vorgesehen ist, die mit einer übermäßigen Proliferation von Hautzellen und insbesondere der Keratinozyten verbunden sind.

14. Verwendung nach einem der Ansprüche 12 und 13, **dadurch gekennzeichnet, daß** die dermatologischen Störungen, die mit einer übermäßigen Proliferation von Hautzellen verbunden sind, ausgewählt sind unter den Hautkrankheiten, die mit einer Störung der Keratinisierung verbunden sind, die die Zellproliferation betrifft, insbesondere der Akne vulgaris, Akne comedonica, der polymorphen Akne, der Akne rosaceae, der nodulären Akne, der Akne conglobata, der altersbedingten Aknen, den als Nebenwirkung auftretenden Aknen, wie der Akne solaris, der medikamentenbedingten Akne oder der Akne professionalis, den sonstigen Störungen der Keratinisierung, insbesondere den Ichthyosen, den ichthyosiformen Zuständen, der Darrier-Krankheit, der Keratosis palmoplantaris, den Leukoplasien und den leukoplasiformen Zuständen, den Haut- und Schleimhautflechten (buccal) (Lichen), den sonstigen Hauterkrankungen, die mit einer Störung der Keratinisierung zusammenhängen und eine entzündliche und/oder immunoallergische Komponente haben und insbesondere allen Formen der Psoriasis, die die Haut, die Schleimhäute und die Finger- und Zehennägel betreffen, und dem psoriatischen Rheuma und den Hautatopien, wie Ekzemen oder der respiratorischen Atopie oder auch der Hypertrophie des Zahnfleischs, allen gutartigen oder bösartigen Wucherungen der Dermis oder Epidermis, die gegebenenfalls viralen Ursprungs sind, wie Verruca vulgaris, Verruca plana, Epidermodysplasia verruciformis, orale Papillomatose, Papillomatosis florida, und den Wucherungen, die durch UV-Strahlung hervorgerufen werden können, insbesondere im Fall es Epithelioma basocellulare und des Epithelioma spinocellulare,

15. Produkt, das mindestens einen Liganden, der eine Wirksamkeit bzgl. der nukleären Rezeptoren vom VDR-Typ aufweist, und mindestens ein Retinoid, das im Vergleich mit RARα-Rezeptoren selektiv für RARγ-Rezeptoren ist, enthält, die wie in den Ansprüchen 1 bis 7 definiert sind, als Kombinationsprodukt für die gleichzeitige, getrennte oder zeitlich verlängerte Verwendung zur Behandlung von Störungen, die mit einer übermäßigen Zellproliferation verbunden sind, insbesondere von Hautkrankheiten, die mit einer übermäßigen Proliferation von Hautzellen und insbesondere der Keratinozyten verbunden sind.

## Claims

1. Combination product consisting of the combination of at least one ligand having activity for nuclear receptors of VDR type and at least one retinoid which is selective for RARγ receptors relative to RARα receptors chosen from 6-[3-(1-adamantyl)-4-hydroxyphenyl]-2-naphthoic acid, 6-[3-(1-adamantyl)-4-methoxyphenyl]-2-naphthoic acid and 2-hydroxy-4-[3-hydroxy-3-(5,6,7,8-tetrahydro-5,5,8,8-tetramethyl-2-naphthyl)-1-propynyl]benzoic acid or derivatives thereof.

2. Product according to Claim 1, for use as a medicinal product.

3. Product according to either of Claims 1 and 2, **characterized in that** the retinoid which is selective for RARγ receptors relative to RARα receptors has an RARα/RARγ dissociation constant ratio greater than or equal to 8.

4. Product according to one of Claims 1 to 3, **characterized in that** the ligands having activity for the VDR receptor are chosen from: vitamin D3, vitamin D2, 25-hydroxyvitamin D3, 1α-hydroxyvitamin D3, 1α,25-dihydroxyvitamin D3 (calcitriol), 1α,25,26-trihydroxyvitamin D3, 1α,23,25-trihydroxyvitamin D3, 24,25-dihydroxyvitamin D3, 1α,25-dihydroxyvitamin D2, 1α-hydroxyvitamin D2, 1α,24-dihydroxyvitamin D2, 1α,24-dihydroxyvitamin D3 (tacalcitol), (5Z,7E,23S)-26,26, 26,27,27,27-hexafluoro-9,10-secocholesta-5,7,10(19)-triene-1α-3β,23,25-tetraol and 26,26,26,27,27,27-hexafluoro-9,10-secocholesta-5,7,10(19)-triene-1α,25-diol.

5. Product according to Claim 4, **characterized in that** the ligand having activity for the VDR receptor is 1α,25-dihydroxyvitamin D3.

6. Product according to one of Claims 1 to 5, **characterized in that** the weight ratio between at least one ligand having activity for the nuclear receptors of VDR type and at least one retinoid which is selective for RARγ receptors relative to RARα receptors is between 1/1000 and 1000/l.

7. Product according to Claim 6, **characterized in that** the weight ratio is between 1/10 and 10/l.

8. Pharmaceutical composition, **characterized in that** it comprises, in a pharmaceutically acceptable support, at least one ligand having activity for nuclear receptors of VDR type and at least one retinoid which is selective for RARγ receptors relative to RARα receptors, as defined in Claims 1 to 7.

9. Composition according to Claim 8, **characterized in that** it is a composition packaged in a form which is suitable for enteral, parenteral, topical or ocular use.

10. Composition according to either of Claims 8 and 9, **characterized in that** the ligand having activity for the nuclear receptors of VDR type is present at a concentration of between 0.001% and 10% by weight, preferably between 0.1 and 1% by weight, relative to the total weight of the composition.

11. Composition according to one of Claims 8 to 10, **characterized in that** the retinoid is present at a concentration of between 0.001% and 10% by weight, preferably between 0.1 and 1% by weight, relative to the total weight of the composition.

12. Use of at least one retinoid which is selective for RARγ receptors relative to RARα receptors chosen from 6-[3-(1-adamantyl)-4-hydroxyphenyl]-2-naphthoic acid, 6-[3-(1-adamantyl)-4-methoxyphenyl]-2-naphthoic acid and 2-hydroxy-4-[3-hydroxy-3-(5,6,7,8-tetrahydro-5,5,8,8-tetramethyl-2-naphthyl)-1-propynyl]benzoic acid or derivatives thereof, for the manufacture of a pharmaceutical composition intended to increase the inhibitory activity on cell proliferation due to at least one ligand having activity for the VDR receptor used, for the treatment of disorders associated with hyperproliferation of skin cells, and more particularly of keratinocytes.

13. Use of the combination product as defined in Claims 1 to 7 for the manufacture of a pharmaceutical composition intended to treat disorders associated with hyperproliferation of skin cells, and more particularly of keratinocytes.

14. Use according to either of Claims 12 and 13, **characterized in that** the dermatological disorders associated with hyperproliferation of skin cells are chosen from dermatological complaints associated with a keratinization disorder which has a bearing on cell proliferation, in particular common acne, comedones, polymorphonuclear leukocytes, acne rosacea, nodulocystic acne, acne conglobata, senile acne, secondary acne such as solar, medication-related or profession-related acne, other keratinization disorders, in particular ichtyosis, ichtyosiform states, Darier's disease, palmoplantar keratoderma, leucoplasia and leucoplasiform states, cutaneous or mucous (buccal) lichen, other dermatological complaints associated with a keratinization disorder with an inflammatory and/or immunoallergic component and, in particular, all forms of psoriasis, whether cutaneous, mucous or ungual psoriasis, and even psoriatic rheumatism, or alternatively cutaneous atopy, such as eczema or respiratory atopy or alternatively gingival hypertrophy, all dermal or epidermal proliferations, whether benign or malignant, and whether of viral origin or otherwise, such as common warts, flat warts and verruciform epidermodysplasia, oral or florid papillomatoses and proliferations which may be induced by ultraviolet radiation, in particular in the case of basocellular and spinocellular epithelioma.

15. Product comprising at least one ligand having activity for nuclear receptors of VDR type and at least one retinoid which is selective for RARγ receptors relative to RARα receptors, as defined in Claims 1 to 7, as a combination product for simultaneous or separate use or use spread out over time, for the treatment of disorders associated with cell hyperproliferation, in particular dermatological disorders associated with hyperproliferation of skin cells, more particularly of keratinocytes.
